# EUROPÄISCHE PATENTSCHRIFT

(11) **EP 3 003 229 B1**
(45) Veröffentlichungstag und Bekanntmachung des Hinweises auf die Patenterteilung: **20.02.2019**
(21) Anmeldenummer: 14728447.5
(22) Anmeldetag: 30.05.2014
(51) Int. Cl.: A61F 2/78, A61F 2/80

(54) **PROTHESENLINER UND PROTHESENSCHAFTSYSTEM MIT PROTHESENLINER UND PROTHESENSCHAFT**
PROSTHETIC LINER AND PROSTHETIC SYSTEM COMPRISING A PROSTHETIC LINER AND PROSTHETIC SOCKET
REVÊTEMENT DE PROTHÈSE ET SYSTÈME DE TIGE DE PROTHÈSE PRÉSENTANT UN REVÊTEMENT DE PROTHÈSE ET UNE TIGE DE PROTHÈSE

(30) Priorität: 03.06.2013 DE 102013009196
(43) Veröffentlichungstag der Anmeldung: 13.04.2016
(73) Patentinhaber: Ottobock SE & Co. KGaA, 37115 Duderstadt (DE)
(72) Erfinder: MÜLLER, André, 37115 Duderstadt (DE)
(74) Vertreter: Stornebel, Kai
(86) Internationale Anmeldenummer: PCT/EP2014/001461
(87) Internationale Veröffentlichungsnummer: WO 2014/194998

(56) Entgegenhaltungen:
- WO-A1-2014/032802
- DE-A1-102004 056 775
- GB-A- 2 486 817
- US-A1- 2012 191 217
- US-B1- 6 454 812

## Beschreibung

Die Erfindung betrifft einen Prothesenliner zur Anlage an einen Stumpf, mit einem elastischen Grundkörper, der eine proximale Öffnung zum Einführen des Stumpfes in einen Aufnahmeraum und ein distales Ende aufweist, sowie ein Prothesenschaftsystem mit einem solchen Prothesenliner und einem Prothesenschaft.

Prothesen dienen zum Ersetzen der Funktion und gegebenenfalls der optischen Erscheinung einer fehlenden Gliedmaße und werden an dem Körper des Patienten festgelegt. Zur Festlegung der Prothese sind vielfältige Möglichkeiten bekannt. Eine weit verbreitete Möglichkeit zur Festlegung von Prothesen an den Extremitäten ist die Anordnung an einem verbliebenen Teil der Gliedmaße, dem sogenannten Stumpf. Der Stumpf wird von einem Prothesenschaft umfasst, der in der Regel formstabil ist. An dem proximalen Ende des Schafts ist eine Einführöffnung ausgebildet, an dem distalen Ende des Schaftes ist zumindest eine Befestigungseinrichtung vorgesehen, an der weitere Prothesenkomponenten angeordnet werden können, beispielsweise Gelenke oder funktionale Einheiten wie Prothesenfüße oder Prothesenhände. Um eine gute Passform zu erreichen, wird ein Modell des Stumpfes angefertigt und der Schaft an die Kontur des Stumpfmodells angepasst. Um Volumenschwankungen ausgleichen zu können, kann vorgesehen sein, dass der Schaft enger gefertigt wird als das abgeformte Stumpfmodell.

Um den Tragekomfort zu erhöhen kann es vorgesehen sein, dass zwischen dem Prothesenschaft und dem Stumpf ein sogenannter Prothesenliner angeordnet ist. Der Prothesenliner besteht in der Regel aus einem geschlossenen Grundkörper mit einer proximalen Öffnung und wird strumpfartig über den Stumpf gezogen. Das elastische Material haftet auf der Stumpfoberfläche und stellt die Verbindung zwischen dem Stumpf und dem Prothesenschaft her. Zur Befestigung des Prothesenliners an dem Prothesenschaft können mechanische Verriegelungselemente am distalen Ende des Prothesenliners und korrespondierende Verriegelungseinrichtungen am distalen Ende des Prothesenschaftes vorgesehen sein, die den Prothesenliner mit dem Prothesenschaft nach dem Einsteigen in den Prothesenschaft formschlüssig verriegeln. Über eine Entriegelungseinrichtung kann die Befestigung des Prothesenliners an dem Prothesenschaft aufgehoben werden.

Eine weitere Möglichkeit zur Befestigung eines Prothesenschaftes an einem Stumpf besteht in der sogenannten Saugschaftlinertechnologie, bei der der Prothesenschaft luftdicht gegenüber dem Prothesenliner abgedichtet wird und aus dem Zwischenraum zwischen dem Prothesenliner und dem Prothesenschaft die darin vorhandene Luft abgesaugt oder herausgedrückt wird. Ein Rückströmen wird über ein Rückschlagventil verhindert. Hierzu ist es notwendig, den Prothesenschaft luftdicht auszugestalten und eine großflächige Abdichtung gegenüber dem Prothesenliner sicherzustellen.

Die US 2011/0035027 A1 betrifft ein vakuumunterstütztes Linersystem mit einem flexiblen Liner, der über einen Stumpf gezogen wird und zumindest eine umlaufende Kante aufweist. Der Liner ist aus einem luftundurchlässigen Material gefertigt und hat zumindest einen porösen Bereich, der von der umlaufenden Kante beabstandet ist, um den Transport von Luft und Feuchtigkeit zwischen einer äußeren Oberfläche und einer inneren Oberfläche des Liners zu ermöglichen.

Die US 2012/0191217 A1 betrifft ein Schaftsystem mit einem Vakuumliner für prothetische oder orthetische Vorrichtungen. An dem geschlossenen Liner ist an dem distalen Ende ein Bereich aus einem elastischen Material angeordnet, das härter als das Material des übrigen Liners ist. Der Bereich weist einen konkaven Abschnitt auf, der sich von einer äußeren Endoberfläche des Bereiches nach innen erstreckt. Der distale Bereich und der konkave Abschnitt bilden zumindest einen Teil einer Vakuumpumpe aus, um Luft aus dem Zwischenraum zwischen dem Liner und dem Prothesenschaft herauszupumpen.

Die US 8,197,555 B1 betrifft eine Vakuumpumpe, die in einem Prothesenschaft an dessen distalem Ende integriert ist. Die Vakuumpumpe besteht aus einem Elastomergehäuse mit zwei Rückschlagventilen und einem Federelement aus einem elastomeren Werkstoff. Wird das Federelement belastet, wird Luft durch ein erstes Rückschlagventil in einen Zwischenraum und aus einem Auslassventil aus dem Zwischenraum in die Umgebung gedrückt. Wird das elastomere Federelement entlastet, wird das Auslassventil geschlossen und das erste Rückschlagventil geöffnet und weitere Luft aus dem Prothesenschaft in den Zwischenraum gesaugt. Die in dem Zwischenraum zwischen dem Prothesenschaft und dem Prothesenliner befindliche Luft wird dadurch herausgepumpt.

Die US 6,979,355 B1 betrifft eine Ventilanordnung für eine Protheseneinrichtung mit einem Prothesenschaft, an dessen distalem Ende ein Kanal ausgebildet ist, der mit einem Ventil verschlossen ist.

Die US 6,063,125 betrifft eine Befestigungseinrichtung für prothetische Gliedmaßen mit einem Schaft und einem distalen Adapter, in dem eine Durchgangsbohrung angeordnet ist. Ein Rückschlagventil ist innerhalb der Bohrung ausgebildet.

Die DE 10 2006 054 981 A1 betrifft einen Prothesenschaft mit aktivem Luftausstoß, bei dem in einer Seitenwand eines Prothesenschaftes eine Pumpblase angeordnet ist, die über ein Ventil mit der Außenumgebung gekoppelt ist.

Die US 2008/0004716 A1 betrifft einen Schaft und einen Liner zur Befestigung an einem Stumpf. In dem Schaft ist ein Rückschlagventil ausgebildet, um Luft aus dem Schaft abzutransportieren.

Die DE 10 2004 056 775 A1 betrifft eine Vorrichtung zum lösbaren Verbinden eines Prothesenschaftes mit einem Prothesenunterteil. An dem distalen Ende des Prothesenschaftes ist ein federnd gelagerter Kolben in einer Kolbenkammer geführt. Eine Dichtung kann an dem Kolben angeordnet sein, um in Verbindung mit einem Ventil in der Kolbenkammer einen Unterdruck zu erzeugen. Zur Begrenzung der Kolbenbewegung sind elastisch vorgespannte Einrastmittel dem Kolben zugeordnet, die in am Kolben ausgebildete Einrastausnehmungen einrasten.

Die US 6 454 812 B1 wird als nächstliegender Stand der Technik angesehen und betrifft einen Prothesenliner mit einer umlaufenden Seitenwand und einem geschlossenen distalen Ende, in dem ein Schirm eingebettet ist, von dem aus sich ein Bolzen in distaler Richtung erstreckt, der aus dem Liner herausragt. Innerhalb des Bolzens ist eine Bohrung eingearbeitet, die mit einem Innengewinde versehen ist, um darüber den Liner mit dem Prothesenschaft zu verschrauben.

Aufgabe der vorliegenden Erfindung ist es, einen Prothesenliner und ein Prothesenschaftsystem bereitzustellen, mit dem eine sichere Befestigung der Prothese an dem Liner bei passiven Vakuumschäften möglich ist und ein verbessertes Haltegefühl sowie ein verbesserter Tragekomfort für den Anwender bereitgestellt werden kann.

Erfindungsgemäß wird diese Aufgabe durch einen Prothesenliner mit den Merkmalen des Hauptanspruchs und ein Prothesenschaftsystem mit den Merkmalen des nebengeordneten Anspruchs gelöst. Vorteilhafte Ausgestaltungen und Weiterbildungen der Erfindung sind in den Unteransprüchen, den Figuren und der Beschreibung offenbart.

Der Prothesenliner zur Anlage an einen Stumpf, mit einem elastischen Grundkörper, der eine Proximalöffnung zum Einführen des Stumpfes in einen Aufnahmeraum und distales Ende aufweist, sieht vor, dass an der Außenseite des Prothesenliners ein Pneumatikkolben angeordnet ist.

Bei einem passiven Vakuumsystem, also einem System, bei dem ein Vakuum zwischen einem Prothesenliner und einem Prothesenschaft durch den Prothesenträger selbst und die Relativbewegung des Prothesenliners relativ zum Prothesenschaft erzeugt wird, wird ein Unterdruck im Schaftinnenraum in der Schwungphase erzeugt, in der die Protheseneinrichtung aufgrund der Fliehkräfte von dem Stumpf wegbewegt wird. Durch den Unterdruck im Schaftinnenraum während der Schwungphase wird eine Haltekraft erzeugt, die für die befestigende Wirkung des Prothesenliners in dem Prothesenschaft verantwortlich ist. Diese Bewegung kann von Anwendern als unangenehm empfunden werden, weil das Abheben des Prothesenschaftes und des Liners zu einem Gefühl von Instabilität und losem Sitz führen kann. Die Luft aus dem Zwischenraum zwischen Prothesenliner und Prothesenschaft wird durch den Pneumatikkolben evakuiert, was zu einer verbesserten Haltekraft führt. Es entsteht ein selbstregulierendes System, welches darauf ausgelegt ist, den optimalen Halt zwischen dem Prothesenschaft und dem Prothesenliner zu gewährleisten. Der Pneumatikkolben ermöglicht eine präzise Führung des distalen Endes des Liners bei effektiver Pumpwirkung aufgrund der vereinfachten Abdichtbarkeit des durch den Pneumatikkolben abgeschlossenen Volumens.

Eine Weiterbildung der Erfindung sieht vor, dass der Pneumatikkolben ein Sperrelement aufweist, das ein Rückströmen von Luft in Richtung auf den Prothesenliner verhindert. Durch die Anordnung eines Sperrelementes, z.B. eines Ventils oder einer umlaufenden Sperrlippe, in oder an einem am distalen Ende des Prothesenliners angeordneten Pneumatikkolben ist es möglich, die Relativbewegung zwischen dem Prothesenliner und dem Prothesenschaft dazu zu nutzen, den Unterdruck innerhalb des Prothesenschaftes zu erhöhen und dadurch das Abheben des Prothesenliners von der Innenwand des Prothesenschaftes zu reduzieren. Die Luft aus dem Zwischenraum zwischen Prothesenliner und Prothesenschaft wird durch den Kolben hindurch evakuiert, was zu einem kompakten Aufbau führt, bei dem für die Evakuierung wesentlichen Komponenten in dem Pneumatikkolben integriert sind..

Der Pneumatikkolben kann als ein separates Bauteil ausgebildet und an dem Prothesenliner befestigt sein, insbesondere kann der jeweilige Pneumatikkolben reversibel an dem distalen Ende oder im distalen Endbereich des Prothesenliners befestigt sein. Durch eine reversible Befestigung oder Befestigungsmöglichkeit an dem Prothesenliner ist es möglich, unterschiedlich große Pneumatikkolben an dem Liner zu befestigen, um eine Anpassung an die jeweils benötigte Haltekraft oder das benötigte Pumpvolumen ermöglichen zu können. Zur reversiblen Befestigung des Pneumatikkolbens an dem Prothesenliner ist zumindest eine Befestigungseinrichtung an dem Prothesenliner und korrespondierend an dem Pneumatikkolben ausgebildet. Die Befestigungseinrichtung kann beispielsweise ein Schraubsystem, ein Klicksystem mit einer Kugelkopflagerung, eine formschlüssige Verriegelung über Klettverschlüsse oder ein Zapfensystem mit korrespondierender Aufnahme und formschlüssiger Verriegelung ausgebildet sein.

Neben der reversiblen Befestigung des Pneumatikkolbens an dem Liner ist es möglich, diesen auch einstückig mit dem Liner auszubilden oder stoffschlüssig daran festzulegen. Dazu kann der Pneumatikkolben beispielsweise über einen elastischen Schaft an dem Prothesenliner befestigt sein, so dass geringe Fluchtungsfehler zwischen dem Pneumatikkolben und der prothesenschaftseitigen Aufnahme ausgeglichen werden können. Durch eine einteilige Ausgestaltung ist es möglich, auf eine separate Montage und ein separates Kopplungselement zu verzichten. Bei einer elastischen Anbindung des Pneumatikkolbens an dem Prothesenliner wird eine Zusammenführung des Prothesenliners mit dem Prothesenschaft erleichtert, da leichte Schiefstellungen des Pneumatikkolbens durch die Materialelastizität ausgeglichen werden können.

Das dem Pneumatikkolben zugeordnete Sperrelement als Ventil in oder an dem Pneumatikkolben oder als Sperrlippe ausgebildet sein, die ähnlich einer Luftpumpe funktioniert und bei einer Ansaugbewegung Luft in das geschlossene Volumen einströmen lässt, bei einer Ausstoßbewegung sich an den Zylinderbereich anlegt und dadurch eine Abdichtung bewirkt.

Um eine sichere Befestigung des Pneumatikkolbens an dem Prothesenliner gewährleisten zu können und darüber hinaus eine ausreichende Stabilität und Kraftverteilung von dem distalen Ende des Prothesenliners auf den Stumpf zu ermöglichen, ist in einer Weiterbildung der Erfindung am distalen Ende eine formstabile Abschlusskappe vorgesehen, an der der Pneumatikkolben festgelegt wird. Die Festlegung kann über die oben beschriebenen Befestigungseinrichtungen erfolgen. Grundsätzlich ist es auch möglich, dass der Pneumatikkolben an dem distalen Ende des Prothesenliners oder der Abschlusskappe angeformt ist, so dass sich eine sehr kompakte Bauweise ergibt.

Der Pneumatikkolben kann zumindest ein umlaufendes Dichtelement aufweisen, insbesondere einen O-Ring, über das das Volumen zwischen dem Pneumatikkolben und dem Zylinderbereich des Prothesenschaftes abdichtbar ist. Grundsätzlich ist es auch möglich und vorgesehen, dass der Kolben auch ohne Dichtelement in dem Zylinderbereich arbeiten kann, wenn die Passgenauigkeit ausreichend hoch ist.

Eine Weiterbildung der Erfindung sieht vor, dass der Pneumatikkolben beweglich an dem Prothesenliner, z.B. über ein Kugelgelenk oder ein Elastomerelement, befestigt ist. Die bewegliche Befestigung kann dabei unmittelbar an dem Prothesenliner oder an einer daran angeordneten Abschlusskappe erfolgen. Durch ein Kugelgelenk ist es möglich, dass der Pneumatikkolben stets senkrecht innerhalb eines korrespondierenden Zylinderbereiches im Prothesenschaft bewegt werden kann, ohne dass eine Verkantung des Pneumatikkolbens innerhalb des Zylinderbereiches erfolgt. Über das Kugelgelenk kann gleichzeitig eine reversible Verbindung zwischen dem Pneumatikkolben und dem Prothesenliner erfolgen, indem das prothesenlinerseitige Befestigungselement als Kugelkopf ausgebildet ist, auf den ein mit einer entsprechenden Aufnahme versehener Pneumatikkolben aufgeclipst wird. Neben einer Lagerung mittels eines Kugelgelenkes, das eine Verdrehung um einen Drehpunkt in der Regel ohne Rückstellkräfte ermöglicht, ist eine elastische Anbindung des Pneumatikkolbens an dem Prothesenliner möglich, beispielsweise über einen elastischen Steg, Zapfen, Bolzen oder dergleichen. Durch die elastische Anbindung ist neben einer Relativverlagerbarkeit des Pneumatikkolbens zu dem Prothesenliner auch eine Führung und Zentrierung innerhalb des Prothesenschaftes gegeben.

Eine Weiterbildung der Erfindung sieht vor, dass der Pneumatikkolben über zumindest einen Magneten an dem Prothesenliner befestigt ist. Über die magnetische, also kraftschlüssige Befestigung des Pneumatikkolbens an dem Prothesenliner, beispielsweise an einem Zapfen einer Abschlusskappe ist es möglich, unterschiedliche Prothesenliner einfach an dem Pneumatikkolben anzuschließen, so dass eine standardisierte Pneumatikeinheit mit unterschiedlichen Prothesenlinern oder umgekehrt gekoppelt werden kann. Auch ist es möglich, durch die magnetische Kopplung eine vergleichsweise einfache Auswechslung einzelner Komponenten vorzunehmen.

Der Pneumatikkolben ist in einer Ausgestaltung der Erfindung über zumindest einen Magneten konnektierbar in einem Prothesenschaftsystem aus dem Prothesenliner, insbesondere wie er oben beschrieben wurde, und einem Prothesenschaft integriert oder integrierbar.

Um bei einer magnetischen Anbindung des Pneumatikkolbens an dem Prothesenliner eine dem Kugelgelenk ähnliche Verschieblichkeit zuzulassen, so dass der Pneumatikkolben selbst stets gradlinig in dem Aufnahmeteil, insbesondere in dem zylindrischen Bereich des Aufnahmeteils geführt werden kann, ist an dem Pneumatikkolben zumindest bereichsweise eine sphärische Oberfläche vorgesehen, die entweder konvex oder konkav ausgebildet sein kann. Über die sphärische Oberfläche ist eine Verlagerbarkeit und gewisse Verschiebbarkeit aufgrund der magnetischen Anbindung möglich, so dass Drehkräfte nicht unmittelbar auf den Pneumatikkolben übertragen werden, so dass eine Verkantung nicht nur oder in Extremsituationen stattfinden kann.

An dem Prothesenliner kann ein Befestigungselement angeordnet sein, über das der Pneumatikkolben an dem Prothesenliner befestigt wird und dass eine zu einer zumindest bereichsweise sphärischen Oberfläche des Pneumatikkolbens korrespondierend ausgebildete Kontaktoberfläche ausweist. Durch die konvex ausgebildete Kontaktoberfläche bei einer konkav ausgebildeten sphärischen Oberfläche des Pneumatikkolbens oder umgekehrt ist es möglich, eine kugelgelenkartige Bewegung des Pneumatikkolbens um das Befestigungselement auszuführen, so dass eine Relativverlagerung des Prothesenliners zu dem Pneumatikkolben hinsichtlich der möglichen Drehachsen stets gegeben ist.

Die Verbindung zwischen dem Prothesenliner und dem Pneumatikkolben kann beim Einsteigen des Nutzers in den Prothesenschaft erfolgen, so dass sobald der Prothesennutzer in den Prothesenschaft einsteigt, der Prothesenliner oder das Befestigungselement die Kontaktfläche in den Kontakt mit den Magneten an oder in dem Pneumatikkolben bringt. Selbst wenn der Kolben im Ruhezustand in der unteren Position befindlich ist, reichen in der Regel die Magnetkräfte aus, um den Kolben wieder in die obere Position zu bewegen. In der Regel befindet sich der Pneumatikkolben jedoch in der oberen Position, da dort der Kolben beim Ausstieg aus dem Prothesenschaft befindlich ist. Eine Trennung des Prothesenliners von dem Pneumatikkolben kann bei einem Ausstieg des Prothesennutzers aus dem Prothesenschaft leicht durch eine Auszugsbewegung erfolgen, die lediglich eine Kraft aufbringen muss, die größer als die der Haltemagnete in dem Pneumatikkolben oder in dem Befestigungselement ausgeübt wird.

Das Prothesenschaftsystem mit einem Prothesenliner zur Anlage an einen Stumpf, mit einem elastischen Grundkörper, der eine proximale Öffnung zum Einführen des Stumpfes in einen Aufnahmeraum und ein distales Ende aufweist, und einem Prothesenschaft, der mit dem Prothesenliner einen Zwischenraum ausbildet, sieht vor, dass an der Außenseite des Prothesenliners zumindest ein Pneumatikkolben angeordnet ist und dass der Prothesenschaft einen zu dem Pneumatikkolben korrespondierenden Zylinderbereich aufweist, der zur Aufnahme des Pneumatikkolbens ausgebildet ist und in dem im gekoppelten Zustand des Prothesenliners mit dem Prothesenschaft ein geschlossenes Volumen ausgebildet ist, das mit zumindest einem Sperrelement gekoppelt ist, das ein Rückströmen von Luft in den Zwischenraum verhindert. Der Prothesenliner kann wie er oben beschrieben wurde ausgestaltet sein oder auch ohne integriertes Ventil oder integrierte Sperrlippe bei einer entsprechenden Ausgestaltung des Zylinderbereiches arbeiten. Der korrespondierende Zylinderbereich ist so ausgebildet, dass der Pneumatikkolben an der Innenseite des Prothesenliners entlang eines Dichtelementes entlang gleiten kann, um Luft aus dem geschlossenen Volumen herauszudrücken.

Erfindungsgemäß ist vorgesehen, dass der Pneumatikkolben zusammen mit dem korrespondierenden Zylinderbereich an dem Prothesenschaft ein geschlossenes Volumen ausbildet, das durch ein Sperrelement, insbesondere ein Rückschlagventil, bei einer Volumenvergrößerung, beispielsweise während der Schwungphase, Luft aus dem Bereich zwischen dem Prothesenschaft und dem Prothesenliner ansaugt und während der Standphase, wenn eine Axialbelastung in Richtung auf das distale Ende des Prothesenschaftes erfolgt, durch ein gleichgerichtetes Rückschlagventil, beispielsweise an dem dem Pneumatikkolben gegenüberliegenden Ende des zylindrischen Bereiches, wieder ausgestoßen wird. Das geschlossene Volumen, das aufgrund der Relativverlagerung zwischen dem zylindrischen Bereich und dem Pneumatikkolben variabel ist, stellt das Pumpvolumen dar, das bei einem vollständigen Hub dem maximalen Ausstoßvolumen entspricht. Je größer der Durchmesser des Pneumatikkolbens und je größer der mögliche Hub, desto größer ist das Pumpvolumen und damit der je Hub erreichbare Unterdruck zwischen dem Prothesenschaft und dem Prothesenliner.

Innerhalb des Volumens kann zumindest eine Sperreinrichtung oder zumindest eine strömungstechnische Verbindung zu einer Sperreinrichtung vorhanden sein, die ein Ausströmen von Luft ermöglicht und ein Rückströmen verhindert. Dies ist beispielsweise durch ein Rückschlagventil möglich, das gegebenenfalls auch geöffnet werden kann, um den Unterdruck zu reduzieren und die Haltekraft zu verringern. Durch das Anordnen einer Sperreinrichtung ist ein nahezu geräuschloses Evakuieren möglich, darüber hinaus kann derjenige Ort definiert werden, an dem die Luft aus dem Volumen herausgedrückt wird. Die Sperreinrichtung oder Sperreinrichtungen ermöglichen ein Absaugen der Luft aus dem Zwischenraum zwischen dem Prothesenliner und dem Prothesenschaft und verhindern ein Rückströmen von Luft aus der Umgebung durch das geschlossene Volumen in den Zwischenraum.

Das geschlossene Volumen und/oder der Zwischenraum können mit einer Belüftungseinrichtung zum Einleiten von Luft in strömungstechnischer Verbindung stehen, um den Unterdruck in dem geschlossenen Volumen oder Zwischenraum aufzuheben und Umgebungsluft in das geschlossene Volumen und/oder den Zwischenraum zwischen dem Prothesenschaft und dem Prothesenliner einleiten zu können, damit der Prothesenliner aus dem Prothesenschaft entfernt werden kann. Die Belüftungseinrichtung weist einen Zugang zu dem geschlossenen Volumen und/oder direkt zu dem Zwischenraum von Prothesenliner und Prothesenschaft auf, um ein Lösen der Verbindung zu ermöglichen. Alternativ zu der Verwendung einer Belüftungseinrichtung kann der Zwischenraum dadurch belüftet werden, dass eine Dichtmanschette, die als proximale Abdichtung eingesetzt wird, entfernt wird. Sofern eine Dichtung in dem Schaft angeordnet oder an dem Liner angeordnet ist, ist eine separate Belüftungseinrichtung notwendig.

An dem proximalen Ende des Zylinderbereiches kann eine Einführschräge vorgesehen sein, die ein Einführen des Pneumatikkolbens in den Zylinderbereich erleichtert, so dass die korrekte Zuordnung zwischen dem Stumpf mit Prothesenliner und dem Prothesenschaft einfach hergestellt werden kann. Über die Einführschräge wird der Pneumatikkolben automatisch zu dem Zylinderbereich geführt und sorgt dort für eine Zentrierung bzw. Orientierung des Prothesenliners innerhalb des Prothesenschaftes.

An dem proximalen Ende des Zylinderbereiches kann eine Auszugssicherung für den Pneumatikkolben angeordnet sein, so dass verhindert wird, dass der Pneumatikkolben bei einem Ausstieg aus dem Prothesenschaft aus dem zylindrischen Bereich herausgezogen wird. Die Auszugssicherung kann mechanisch, insbesondere formschlüssig an dem Aufnahmeteil angeordnet sein, beispielsweise eingeschraubt, eingeklemmt oder eingeclipst. Vorteilhafterweise ist eine umlaufende Ausgestaltung der Auszugssicherung durch einen angeschraubten oder anderweitig festgelegten Ring vorgesehen. Nachfolgend werden Ausführungsbeispiele der Erfindung anhand der beigefügten Figuren näher erläutert. Gleiche Bezugszeichen bezeichnen gleiche Komponenten. Es zeigen:
- Figur 1: eine schematische Darstellung eines Prothesenliners und eines Pneumatikkolbens in Seitenansicht;
- Figur 2: eine Schnittansicht eines Pneumatikkolbens;
- Figur 3: eine perspektivische Ansicht eins Pneumatikkolbens;
- Figur 4: eine Schnittansicht durch einen Prothesenliner während des Fügens;
- Figur 5: eine perspektivische Ansicht eines Aufnahmeteils;
- Figur 6: eine Schnittdarstellung im montierten Zustand;
- Figur 7: eine schematische Ansicht einer Variante;
- Figur 8: eine Detailansicht der Figur 7;
- Figur 9: eine Einzeldarstellung von dem Aufnahmeteil mit Pneumatikkolben und Abschlusskappe;
- Figur 10: eine schematische Schnittansicht in einer ersten Variante;
- Figur 11: eine schematische Schnittansicht im montierten Zustand des Prothesenliners;
- Figur 12: eine Darstellung einer Kippposition mit Auszugssicherung;
- Figur 13: eine Darstellung der Figur 12 in einer abgesenkten Position;
- Figur 14: eine Darstellung der Figur 13 in der tiefsten Endposition;
- Figur 15: eine perspektivische Darstellung der Position gemäß Figur 12;
- Figur 16: eine Schräg-Unten-Ansicht der Position gemäß Figur 10;
- Figur 17: eine Schrägansicht der Position gemäß Figur 16;
- Figur 18: eine Seitenansicht der Position gemäß Figur 17;
- Figur 19: eine Schrägdraufsicht auf ein Aufnahmeteil mit eingeführtem Pneumatikkolben und Auszugssicherung;
- Figur 20: eine Einzeldarstellung eines Pneumatikkolbens;
- Figur 21: eine Schräg-Unten-Ansicht des Pneumatikkolbens gemäß Figur 20;
- Figur 22: eine Schräg-Unten-Ansicht einer Abschlusskappe und eines Pneumatikkolbens; sowie
- Figur 23: eine Seitenansicht der Stellung gemäß Figur 22.

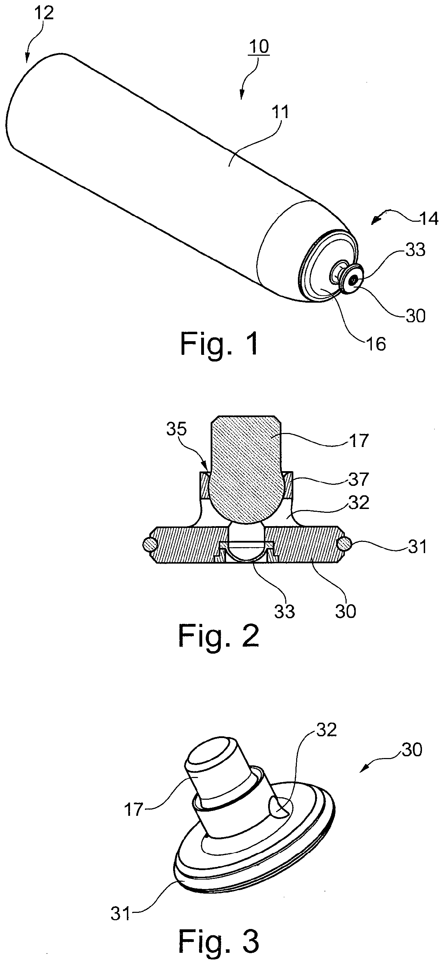

Figur 1 zeigt einen Prothesenliner 10 mit einem elastischen Grundkörper 11, der beispielsweise aus Silikon oder einem anderen Elastomer hergestellt sein kann. An dem proximalen Ende 12 des Prothesenliners ist eine Öffnung vorhanden, durch die ein Prothesennutzer in einen Aufnahmeraum innerhalb des Prothesenliners 10 einsteigen kann. Der Aufnahmeraum ist in dieser Figur nicht gezeigt. Das Anlegen des Prothesenliners 10 erfolgt in der Regel durch Herabrollen des proximalen Endes 12 oder Umstülpen des proximalen Endes 12, Anlegen eines distalen Endes 14 des Prothesenliners an das distale Ende des Stumpfes und anschließendes Abrollen auf den Stumpf. An dem distalen Ende 14 des Prothesenliners 10 ist im dargestellten Beispiel eine formstabile Abschlusskappe 16 angeordnet, die zur stabilen und sanften Bettung des Stumpfes gegenüber dem Anschluss eines Pneumatikkolbens 30 auf der Außenseite 15, also der dem Aufnahmeraum gegenüberliegenden Seite des Prothesenliners 10, dient. An der Abschlusskappe 16 sind Befestigungseinrichtungen 17 angeordnet, über die es möglich ist, den Pneumatikkolben 30 an der Abschlusskappe 16 anzuordnen. Der Pneumatikkolben 30 verfügt ebenfalls über eine Befestigungseinrichtung 37, über die der Pneumatikkolben 30 an dem Prothesenliner 10, insbesondere an der distalen Abschlusskappe 16, festlegbar ist.

In der Figur 2 ist die Unterseite des Pneumatikkolbens 30 zu erkennen, ebenso ist das Dichtelement 31 am äußeren Umfang des Pneumatikkolbens 30 zu erkennen. Durch die Anordnung des Dichtelementes 31 am äußeren Umfang des Pneumatikkolbens 30 wird eine dichte Anlage an einem Zylinderbereich möglich, so dass bei einer Relativbewegung zwischen dem Pneumatikkolben 30 und dem Zylinderbereich ein Pumpeffekt bewirkt wird. Der Pneumatikkolben 30 ist über die Befestigungselemente 17, 37 reversibel an dem Prothesenliner 10 befestigbar. Dadurch ist es möglich, unterschiedliche Pneumatikkolbentypen an dem Liner festzulegen. Die Pneumatikkolben 30 können sich hinsichtlich Durchmesser, Formgebung oder Materialwahl unterscheiden. Der Pneumatikkolben 30 kann auch anstelle eines Pins eingeschraubt werden, wodurch es möglich ist, einen Standardliner nach der entsprechenden Umrüstung zu verwenden.

Die Kopplung des Pneumatikkolbens 30 mit dem Prothesenliner 10 ist vorzugsweise als eine mechanische Kopplung, beispielsweise als Kugelkopfgelenk 35 ausgeführt, ebenso ist es möglich, dass eine Klebeverbindung zwischen den Komponenten ausgeführt ist. Alternativ zu einer separaten Ausgestaltung des Pneumatikkolbens 30 ist es möglich, diesen direkt in die distale Anschlusskappe 16 zu integrieren, beispielsweise integral anzuformen oder im Rahmen eines 2K-Spritzgussverfahrens anzuspritzen. Die Abschlusskappe 16, die in einem gewissen Maße auch noch verformbar ist, kann aus dem Material des Prothesenliners 10 gefertigt sein und bei einer stoffschlüssigen Verbindung mit dem Pneumatikkolben 30 ein zusammenhängendes System zwischen dem Prothesenliner 10 und dem Pneumatikkolben 30 bilden. Auch ist es möglich und vorgesehen, dass das dem Prothesenliner 10 zugeordnete Befestigungselement 17 einstückig mit dem Prothesenliner 10 ausgebildet ist oder stoffschlüssig damit verbunden ist, beispielsweise an der Abschlusskappe 16 befestigt oder angeformt.

In der Figur 2 ist weiterhin zu erkennen, dass eine strömungstechnische Verbindung 32 in Gestalt einer Querbohrung innerhalb des Pneumatikkolbens 30 an einem zum Prothesenliner 10 hin orientierten Vorsprung oder Absatz ausgebildet ist. Die Querbohrung 32 mündet in eine zur Hauptfläche des Pneumatikkolbens 30 senkrecht orientierte Bohrung, so dass eine strömungstechnische Verbindung 32 von der Umgebungsatmosphäre um den Prothesenliner 10 zur distalen Stirnseite des Pneumatikkolbens 30 vorhanden ist. Innerhalb dieser strömungstechnischen Verbindung 32 ist ein Ventil 33 in Gestalt eines Rückschlagventils angeordnet, das ein Durchströmen in Richtung auf die distale Stirnseite des Pneumatikkolbens 30 zulässt, ein Zurückströmen durch die strömungstechnische Verbindung 32 jedoch sperrt. Dadurch ist es möglich, dass Luft aus der Umgebung des Prothesenliners 10, beispielsweise aus dem Zwischenraum 60 zwischen dem Prothesenliner 10 und einem Prothesenschaft 20, herausgesaugt und in die Umgebung abtransportiert werden kann, ein Eindringen von Luft in diesen Zwischenraum 60 durch die strömungstechnische Verbindung 32 jedoch nicht möglich ist. Es können mehrere Querbohrungen vorhanden sein, so dass die Befestigungselemente 37 als fingerartige Federelemente ausgebildet sein können, um die Kugelgelenklagerung 35 auszubilden.

Neben nur einem Dichtelement 31 an dem äußeren Umfang können auch mehrere Dichtelemente 31 an dem äußeren Umfang des Pneumatikkolbens 30 axial zueinander beabstandet angeordnet sein, um eine verbesserte Führung in einem korrespondierenden zylindrischen Bereich 21 eines Prothesenschaftes 20 zu ermöglichen.

Figur 3 zeigt eine perspektivische Darstellung der Figur 2, die die separate Ausgestaltung des Pneumatikkolbens 30 an dem Befestigungselement 17 zeigt. Das Befestigungselement 17 kann reversibel an dem Prothesenliner 10, insbesondere der Abschlusskappe 16 festlegbar sein.

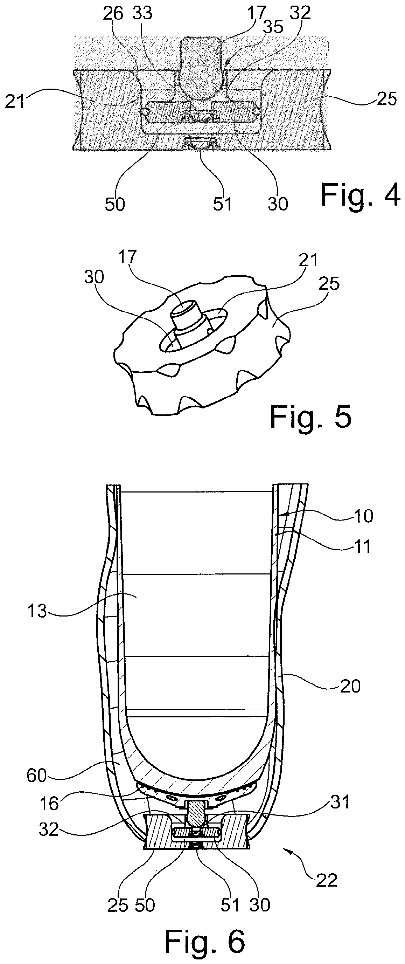

In der Figur 4 ist in einer Schnittdarstellung eine Detailansicht gezeigt, bei der der Pneumatikkolben 30 gemäß Figur 2 in einen Zylinderbereich 21 eines Aufnahmeteils 25 eingeführt ist. Das Aufnahmeteil 25 und der Zylinderbereich 21, der darin ausgebildet ist, bilden das korrespondierende Gegenstück zu dem Pneumatikkolben 30, der mit dem Dichtelement 31 dichtend an der Wand des Zylinderbereiches 21 anliegt. Der Pneumatikkolben 30 verschließt den Zylinderbereich 21 in proximaler Richtung und bildet ein geschlossenes Volumen 50 mit ihm aus, das ein Auslassventil 51 in Gestalt eines Rückschlagventils aufweist, so dass bei einer Relativbewegung zwischen dem Aufnahmeelement 25 und dem Pneumatikkolben 30 in Richtung auf das Auslassventil 51 die in dem geschlossenen Volumen 50 komprimierte Luft herausgedrückt werden kann. Findet dann im Falle einer Entlastung eine Relativbewegung des Pneumatikkolbens von dem Auslassventil 51 weg statt, wird durch die strömungstechnische Verbindung 32 aus dem Umgebungsbereich des nicht dargestellten Prothesenliners 10 Luft durch das Ventil 33 in das geschlossene Volumen 50 hineinbefördert, da sich relativ zu dem geschlossenen Volumen 50 ein Überdruck gebildet hat. Über eine Umkehrbewegung wird dann wieder die im geschlossenen Volumen 50 befindliche und komprimierte Luft über das Ausstoßventil 51 ausgestoßen.

An dem proximalen Rand des Zylinderbereiches 21 ist eine Einführschräge 26 in dem Aufnahmeteil 25 eingearbeitet, die im dargestellten Ausführungsbeispiel einen Radius ausbildet, so dass auch bei einer zunächst ungenauen Positionierung des Prothesenliners 10 in einem Prothesenschaft, beispielsweise bei Einführen, eine zutreffende und gewünschte Zuordnung zwischen dem Pneumatikkolben 30 und dem Zylinderbereich 21 realisiert werden kann.

Durch die in der Figur 4 dargestellte Kombination aus abgedichtetem Pneumatikkolben 30 und Zylinderbereich 21 entsteht eine Kolbenpumpe, über die Luft aus dem Umgebungsbereich des Prothesenliners 10, insbesondere aus dem Zwischenraum zwischen einem als Saugschaft ausgebildeten Prothesenschaft 20 und dem Prothesenliner 10 abgesaugt werden kann. Wird der Saug- und Pumpvorgang mehrmals wiederholt, ändert sich mit jedem Kolbenhub der Unterdruck innerhalb des Prothesenschaftes 20. Aufgrund der Verringerung des Spiels zwischen dem Prothesenliner 10 und dem Prothesenschaft 20 wird der Kolbenhub immer weiter reduziert, da eine Rückkehrbewegung des Pneumatikkolbens 30, also einer Vergrößerung des Volumens 50, immer weiter reduziert wird. Aufgrund der direkten mechanischen Kopplung des Pneumatikkolbens 30 und des Prothesenliners 10 wird solange eine Hubbewegung stattfinden, bis der Prothesenliner 10 keine Bewegung mehr in dem Prothesenschaft 20 durchführt. Ist dieser Zustand erreicht, ist davon auszugehen, dass sich eine optimale Haltekraft zwischen dem Prothesenliner 10 und dem Prothesenschaft 20 eingeregelt hat, da nun der Prothesenliner 10 bündig an der Innenseite des Prothesenschaftes 20 anliegt.

Eine dreidimensionale perspektivische Darstellung des Aufnahmeteiles 25 mit dem eingeführten Pneumatikkolben 30 und dem Befestigungselement 17, das dem Prothesenliner 10 zuzuordnen ist, ist in der Figur 5 dargestellt. Das Aufnahmeteil 25 kann an dem nicht dargestellten Prothesenschaft 20 befestigt sein und aus einem hochgradig formstabilen Werkstoff bestehen, beispielsweise einem Leichtmetall oder einem faserverstärkten Kunststoff, der im Zylinderbereich 21 mit einer glatten Oberfläche ausgestattet ist oder einen Einsatz aufweist, mit dem eine günstige Reibpaarung zwischen dem Pneumatikkolben 30 und dem Zylinderbereich 21 hergestellt werden kann. An dem Aufnahmeteil 25 können mechanische Befestigungselemente oder Aufnahmeeinrichtungen für solche Befestigungselemente angeordnet oder ausgebildet sein, beispielsweise Gewinde, Bohrungen, Hinterschneidungen oder dergleichen. Grundsätzlich ist es auch möglich, das Aufnahmeteil 25 an dem Prothesenschaft 20 über Kleben, Schweißen oder Klettverbindungen zu befestigen.

In der Figur 6 ist in der Schnittdarstellung ein Prothesenliner 10 mit der distalen Abschlusskappe 16 und dem in dem Prothesenschaft 20 eingeführten Pneumatikkolben 30 und die Anwendung des Prothesenliners 10 mit dem an dessen Außenseite 15 angeordneten und daran befestigten, beweglich gelagerten Pneumatikkolben 30 in Verbindung mit einem Prothesenschaft 20 dargestellt. Der Prothesenschaft 20 ist im Wesentlichen der äußeren Kontur des nicht dargestellten Stumpfes nachgebildet und im dargestellten Ausführungsbeispiel aus einem formstabilen luftdichten Werkstoff, beispielsweise einem faserverstärkten Kunststoff, ausgebildet. Der Anwender steigt mit dem Prothesenliner 10 und dem am distalen Ende 14 reversibel befestigten oder einstückig ausgebildeten Pneumatikkolben 30 durch die proximale Öffnung des Prothesenschaftes 20 in diesen hinein. Dadurch wird durch die Aufbringung des Körpergewichtes in Distalrichtung auf den Pneumatikkolben 30 eine Kraft aufgebracht. Auch wenn der Prothesenschaft 20 an dem distalen Ende einen Freiraum aufweist, der eine präzise Einführung in des Pneumatikkolbens 30 in den Zylinderbereich 21 erschwert, kann der Pneumatikkolben 30 aufgrund der Einführschräge 21 sicher in den Zylinderbereich 21 eingeführt werden. Das Befestigungselement 17 kann elastisch ausgebildet sein, beispielsweise aus einem TPE, so dass es in diesem Fall zu einer Verformung des Befestigungselementes 17 beim Einführen des Pneumatikkolbens 30 kommt. Durch das Körpergewicht wird der Pneumatikkolben 30 in dem Zylinderbereich 21 eingeführt und die innerhalb des dabei gebildeten geschlossenen Volumens befindliche Luft durch das Auslassventil 51 herausgedrückt. Das Auslassventil 51 ist als Rückschlagventil oder Ein-Wege-Sperrventil ausgebildet, das Luft oder ein anderes Medium aus dem Volumen 50 herauslässt, ein Eindringen in das Volumen 50 bei einer umgekehrten Bewegung des Pneumatikkolbens 30 jedoch sperrt.

Zwischen der Prothesenlineraußenseite und der Prothesenschaftinnenseite wird daher ein Volumen in dem Zwischenraum 60 aufgespannt, das durch eine wiederholte Kraftaufbringung in Distalrichtung und eine umgekehrte Entlastungsbewegung durch den Pneumatikkolben 30 evakuiert werden kann. Durch den entstehenden Unterdruck z.B. in einer Schwungphase wird zudem eine, wenn auch geringe, Haltekraft zwischen dem Pneumatikkolben 30 und dem Prothesenschaft 20 erzeugt, so dass der Prothesenliner 10, der haftend an der Haut des Stumpfes liegt, über den Pneumatikkolben 30, an dem der Prothesenliner 10 mechanisch gekoppelt ist, an dem Prothesenschaft 20 gehalten und geführt wird. Die Haupthaltekraft wird durch den Unterdruck in dem Zwischenraum 60 zwischen dem Prothesenliner und dem Prothesenschaft bereitgestellt.

In der Schnittdarstellung der Fig. 6 ist auch der Aufnahmeraum 13 für den Stumpf des Prothesennutzers gezeigt. Durch die Druckbewegung in Distalrichtung wird der Pneumatikkolben 30 zusammen mit dem Prothesenliner 10 in Richtung auf das distale Ende 22 des Prothesenschaftes 20 bewegt. Das zwischen dem Pneumatikkolben 30 und dem Zylinderbereich 21 vorhandene geschlossene Volumen 50 wird komprimiert, das innerhalb des Volumens 50 enthaltene Gas, in der Regel Luft, wird herausgedrückt und der Prothesenliner 10 kann weiter in Richtung auf das distale Ende 22 des Prothesenschaftes 20 verschoben werden, bis der Pneumatikkolben 30 die am distalen Ende 22 befindliche Grundfläche der Zylinderbereiches 21 erreicht hat.

Ist die Endposition des Prothesenliners 10 und damit auch des Pneumatikkolbens 30 innerhalb des Prothesenschaftes 20 erreicht und das Volumen 50 minimiert, führt eine Belastung des Prothesenliners 10 in Proximalrichtung zu einer Volumenvergrößerung sowohl des geschlossenen Volumens 50 als auch des Zwischenraumes 60, wodurch ein relativer Unterdruck sowohl zu der Außenumgebung als auch zu dem Zwischenvolumen zwischen dem Prothesenschaft 20 und dem Prothesenliner 10 entsteht. Dieser Unterdruck hält einerseits den Pneumatikkolben 30 in dem Zylinderbereich 21 und saugt andererseits Luft aus dem Zwischenraum 60, die beim Belasten, beispielsweise beim Auftreten, wieder aus dem geschlossenen Volumen 50 herausgedrückt wird. Gegen ein ungewolltes Herausgleiten des Pneumatikkolbens 30 aus dem Zylinderbereich 21 kann eine mechanische Sperre, z.B. ein verstellbarer Anschlag, ein Raststift oder eine andere Art der Bewegungsbegrenzung vorgesehen sein.

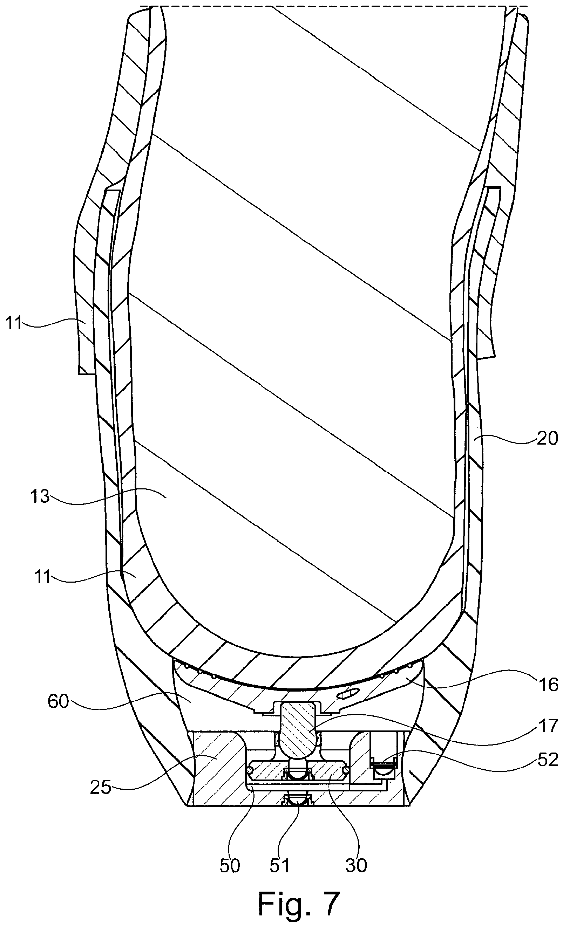

In der Figur 7 ist eine Variante der Erfindung dargestellt, die im Wesentlichen dem Aufbau der der Vorrichtung gemäß Figur 6 entspricht. Der Prothesenliner 10 ist an der Außenseite um den proximalen Rand des Prothesenschaftes 20 umgeschlagen oder mit einer äußeren Hülse versehen, die an der Außenseite des Prothesenliners 10 festgelegt ist und einen Spalt ausbildet, in den der Prothesenschaft 20 eingeführt werden kann, so dass eine Abdichtung auch auf der Außenseite des Prothesenschaftes 20 erfolgt. Der umgeschlagene Prothesenliner 10 oder die daran angeordnete Hülse sind luftdicht ausgebildet, die Hülse kann an dem Prothesenliner 10 angeklebt, angeschweißt oder pressend und haftend aufgelegt sein. Dadurch wird das von dem Prothesenliner 20 und dem Prothesenschaft 10 eingeschlossene Volumen im Zwischenraum 60 sicher beibehalten.

Statt eines Ventils oder einer Sperreinrichtung in dem Pneumatikkolben 30 ist es vorgesehen, dass das Sperrelement 52 nicht in dem Pneumatikkolben 30 angeordnet ist, sondern innerhalb des Aufnahmeteils 25, in dem eine strömungstechnische Verbindung von dem Zwischenraum 60 zu dem geschlossenen Volumen 50 in Gestalt eines Kanals vorhanden ist. Der Kanal verbindet die Saugseite des Pneumatikkolbens 30 mit seiner Druckseite. Innerhalb dieser strömungstechnischen Verbindung ist das Sperrelement 52 in Gestalt eines Rückschlagventils angeordnet, das ein Zurückströmen von Luft von der Druckseite zur Saugseite verhindert. Bei einer Auszugsbewegung des Prothesenliners 11 und damit einer Bewegung des Pneumatikkolbens 30 von der distalen Endposition in Richtung auf die proximale Einführposition, wird in dem geschlossenen Volumen 50 ein Unterdruck erzeugt, durch den Luft aus dem Zwischenraum 60 in das geschlossene Volumen 50 eingesaugt wird. Bei einer Eintauchbewegung des Pneumatikkolbens 30 wird dann die in dem geschlossenen Volumen befindliche Luft durch das Auslassventil 51 herausbefördert.

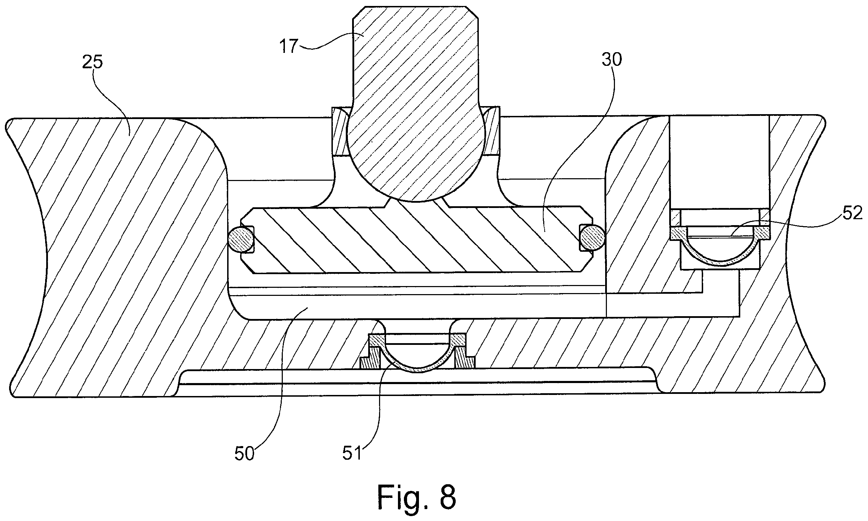

In der Figur 8 ist das Aufnahmeteil 25 mit dem Pneumatikkolben 30 isoliert dargestellt, der Verbindungskanal zwischen dem Zwischenraum und dem geschlossenen Volumen 50 ist über die Sperreinrichtung 52 gegen ein Herausströmen aus dem geschlossenen Volumen gesperrt, so dass ein Abpumpen von Luft aus dem Zwischenraum 60 zwischen dem Prothesenschaft und dem Prothesenliner möglich ist. Die Anordnung eines Rückschlagventils 52 in einem separaten Kanal an dem Aufnahmeteil 25 hat bei kleinen Abmessungen des Pneumatikkolbens 30 Vorteile, da in dem Pneumatikkolben nur ein begrenzter Bauraum zur Verfügung steht.

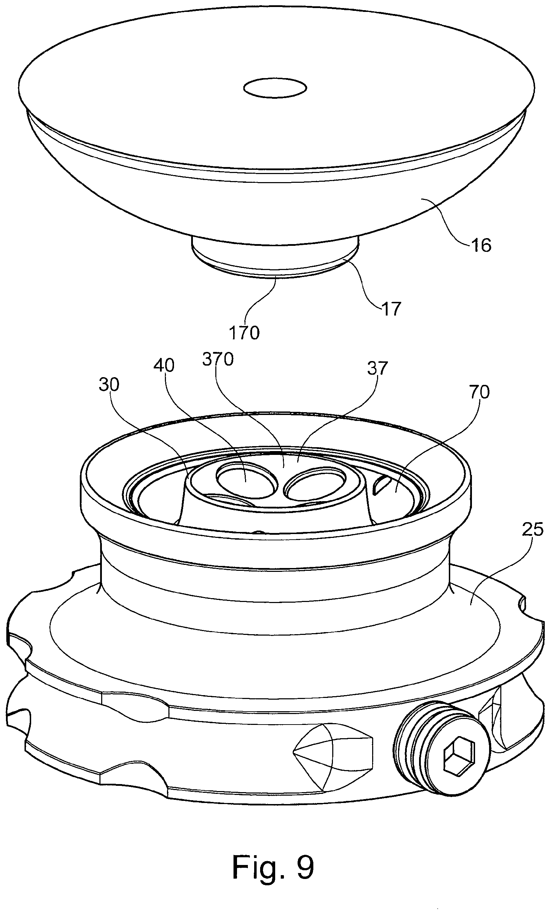

Figur 9 zeigt eine perspektivische Darstellung von Teilen des Prothesenschaftsystems mit einer Abschlusskappe 16, die ohne den dazugehörigen Prothesenliner dargestellt ist. An dem distalen Ende der Anschlusskappe 16 ist die Befestigungseinrichtung 17 in Gestalt eines Befestigungszapfens angeordnet. Das Aufnahmeteil 25 ist isoliert dargestellt, ohne die Einbindung in einen Prothesenschaft. Innerhalb des Aufnahmeteils 25 ist der Pneumatikkolben 30 eingeführt, über das proximale Ende des Pneumatikkolbens 30 ragt aus dem proximalen Ende des zylindrischen Bereiches des Aufnahmeteils 25 heraus. An dem proximalen Rand des Aufnahmeteils 25 ist eine ringförmige Auszugssicherung 70 reversibel angeordnet, die den Durchmesser des zylindrischen Bereiches innerhalb des Aufnahmeteils 25 verringert und verhindert, dass bei einer Auszugsbewegung in proximale Richtung der Kolben 30 aus dem Aufnahmeteil 25 herausgezogen wird.

An dem Kolben 30 ist als Befestigungseinrichtung 37 ein proximaler Endbereich ausgebildet, der eine sphärische Oberfläche 370 aufweist, in der vier Magnete 40 eingelassen sind. Die Magnete 40 sind entweder ebenfalls mit einer sphärischen Oberfläche ausgestattet und schließen bündig mit der sphärischen Oberfläche 370 des Befestigungselementes 37 ab oder sind darin zurückgesetzt eingelassen.

Die Befestigungseinrichtung 17 an der Abschlusskappe 16 weist eine korrespondierend zu der sphärischen Oberfläche 370 geformte Kontaktfläche 170 auf, wobei in der Befestigungseinrichtung 17 entweder ebenfalls Magnete eingelassen sind, die Befestigungseinrichtung 17 als Magnet ausgebildet oder zumindest aus einem magnetischen, insbesondere ferromagnetischem Material besteht, so dass die Befestigungseinrichtung 17 den Magneten 40 innerhalb des Pneumatikkolbens 30 wechselwirken kann. Alternativ dazu ist es möglich, dass der Pneumatikkolben 30 an dem proximalen Ende eine konvex gewölbte, sphärische Oberfläche 370 aufweist und die Kontaktfläche 170 eine korrespondierend dazu geformte konkave Ausgestaltung hat. Auch ist es möglich, dass die Magnete oder nur ein Magnet an dem Befestigungselement 17 des Prothesenliners bzw. der distalen Abschlusskappe 16 angeordnet und der Pneumatikkolben 30 ferromagnetisch ausgebildet ist.

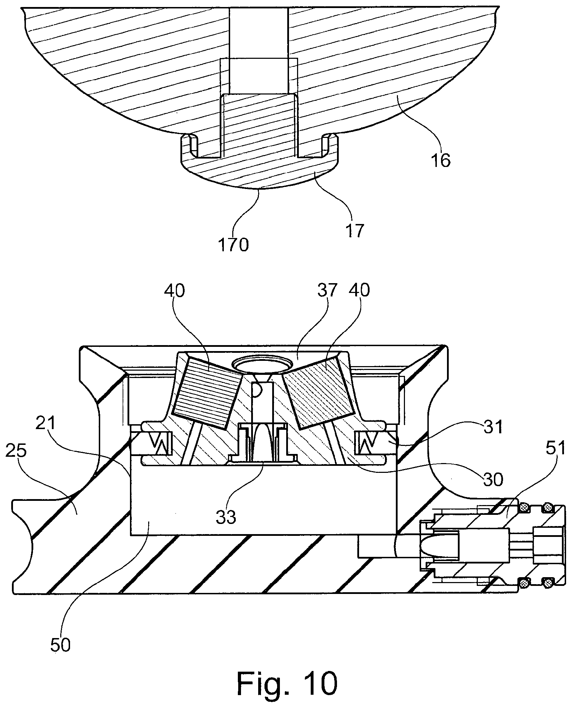

Figur 10 zeigt die Abschlusskappe 16 mit dem darin eingeschraubten Befestigungselement 17 und der sphärisch geformten Kontaktfläche in Schnittdarstellung, in dem Aufnahmeteil 25 ist der zylindrische Bereich 21 ebenso zu erkennen, wie der daran angeordnete Pneumatikkolben 30 mit der radial wirkenden Dichtung 31 und den in dem Pneumatikkolben 30 in der Befestigungseinrichtung 37 gelagerten Magnete 40. Die Magnete 40 sind vorteilhafterweise gleichsinnig gepolt, so dass sie unabhängig von der Drehrichtung oder rotatorischen Orientierung des Pneumatikkolbens 30 innerhalb des Aufnahmeteils 25 eine sichere Zuordnung zu dem Befestigungszapfen 17 und der Kontaktfläche 170 gewährleisten. Alternativ dazu ist es möglich, die Magnete 40 mit einer alternierenden Polung auszustatten und an dem Befestigungselement 17 eine entsprechend anders gepolte Magnetanordnung vorzusehen, so dass eine im Wesentlichen gleiche Ausrichtung von Pneumatikkolben 30 und Prothesenliner 10 nach dem in Eingrifftreten gewährleistet ist.

Unterhalb des Kolbens 30 ist das geschlossene Volumen 50 ausgebildet, innerhalb des Kolbens 30 ist ein Ventil 33 vorgesehen, von dem geschlossenen Volumen 50 zweigt ein Kanal mit einem Auslassventil 51 ab, so dass die Luft aus dem geschlossenen Volumen austreten kann. Durch den Pneumatikkolben 30 kann die Luft aus dem Prothesenschaft oder dem Prothesenliner abgesaugt werden.

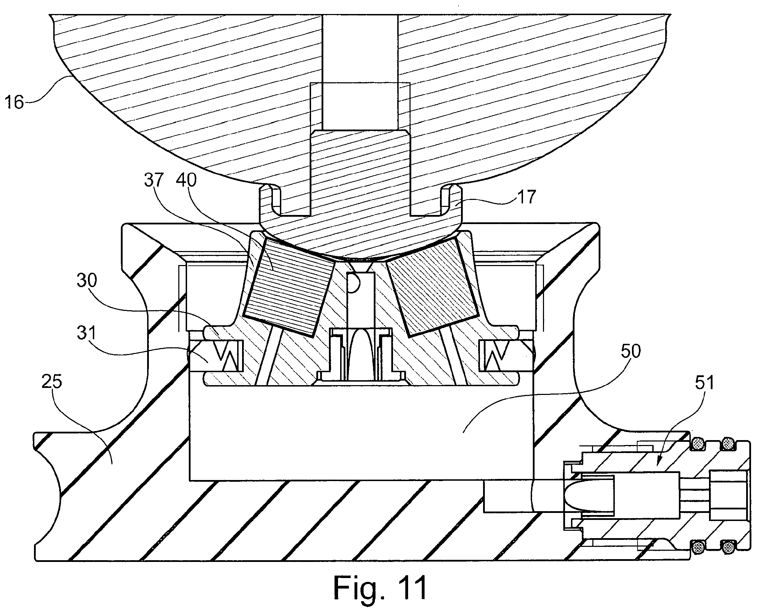

Figur 11 zeigt die Ausgestaltung gemäß Figur 10 im gefügten Zustand. Der Pneumatikkolben 30 befindet sich in der oberen Stellung, die Magnete 40 ziehen das Befestigungselement 17 an den Kolben 30 heran und die Kontaktfläche 170 liegt vollflächig an der sphärischen Oberfläche 370 der kolbenseitigen Befestigungseinrichtung 37 an.

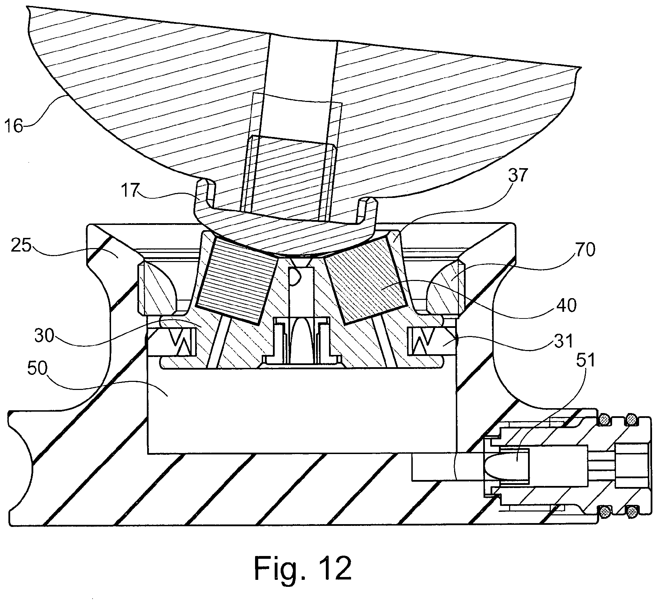

Figur 12 zeigt die Anordnung gemäß Figur 11 mit der montierten Auszugssicherung 70, die in dem Aufnahmeteil 25 angeordnet, beispielsweise eingeschraubt ist. Die Oberkante des Pneumatikkolbens 30 liegt an der Unterkante der Auszugssicherung an, wie dies beispielsweise während einer Schwungphase der Fall ist. Die Abschlusskappe 16 ist leicht zu der lotrechten Achse verkippt, so dass die Kontaktfläche 170 auf der sphärischen Oberfläche 370 verschoben ist, ohne den Kontakt zu verlieren. Aufgrund der gewölbten Oberflächen ist es möglich, dass eine Relativbewegung, insbesondere eine Verkippung um drei Schwenkachsen, die sich im Mittelpunkt der sphärischen Oberfläche 370 schneiden, ohne Probleme möglich ist.

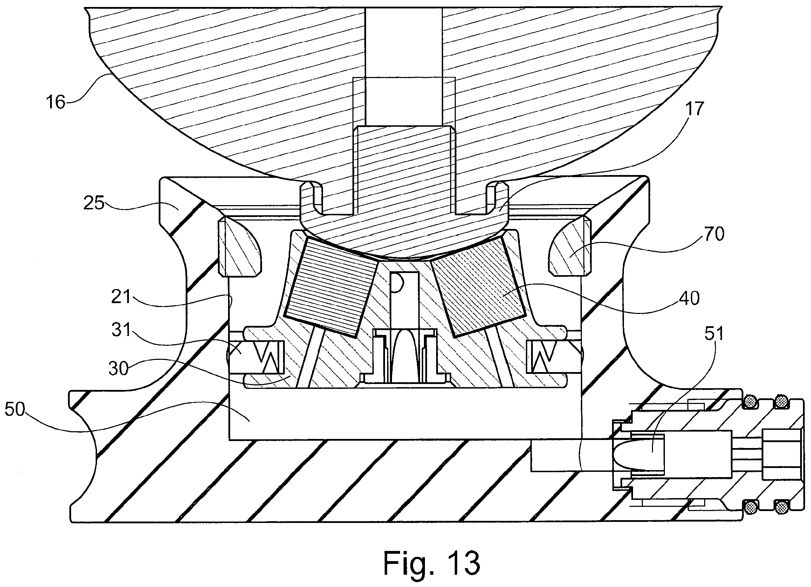

In der Figur 13 ist die Anordnung gemäß Figur 11 zusammen mit der Auszugssicherung 70 in der mittleren, abgesenkten Stellung gezeigt, der Kolben ist nach unten in Richtung distales Ende des zylindrischen Bereiches 21 verlagert, das geschlossene Volumen 50 ist verringert und die Luft ist aus dem Auslassventil 51 herausgedrückt worden.

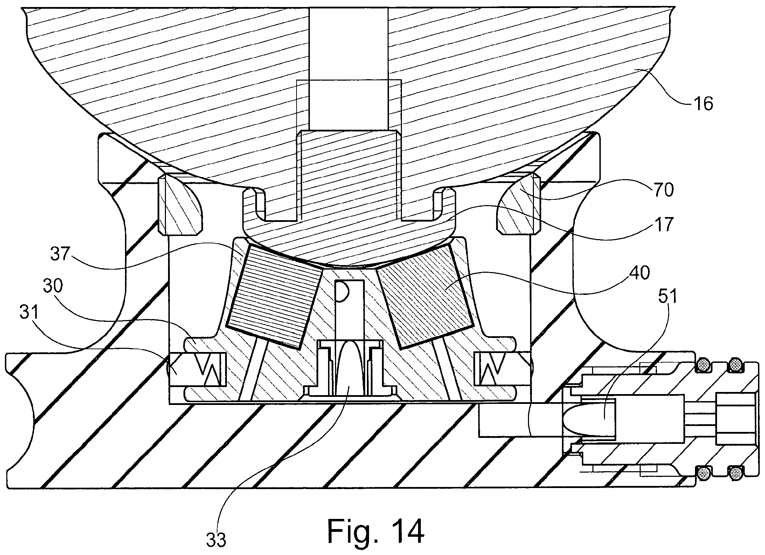

Figur 14 zeigt die Ausgestaltung gemäß Figur 13 in der distalen Endstellung, der Kolben 30 ist nahezu in Berührung mit dem Zylinderboden, das geschlossene Volumen 50 ist minimal. Die Abschlusskappe 16 befindet nahezu in Kontakt mit dem proximalen Ende des Aufnahmeteils 25.

Über die Einschraubhöhe oder Einschraubtiefe des Befestigungselementes 17 in die Abschlusskappe 16 kann der maximale Kolbenhub des Pneumatikkolbens 30 eingestellt werden. Wird der Pneumatikkolben 30 bei einer Entlastung, beispielsweise während der Schwungphase, wieder in Richtung auf die Auszugssicherung 70 verlagert, strömt Luft entweder aus dem Prothesenschaft 20 oder aus dem Prothesenliner 10 durch das Ventil 30 in das sich vergrößernde, geschlossene Volumen 50. Bei einer gegenläufigen Bewegung, beispielsweise während der Standphase, wird das geschlossene Volumen 50 wieder verringert und die angesaugte Luft herausgedrückt.

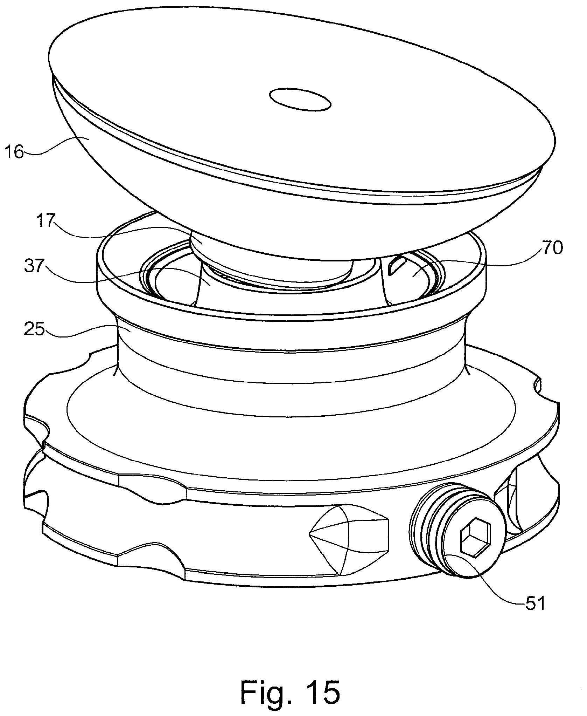

Figur 15 zeigt die Anordnung gemäß Figur 12 in einer Schrägansicht, die Abschlusskappe 16 ist zu der Vertikalachse verkippt, die Auszugssicherung 70 liegt ringförmig um den zylindrischen Bereich 21 des Aufnahmeteils 25 und verhindert, dass bei einer Trennung der kraftschlüssigen Verbindung zwischen dem Prothesenliner 10 und dem Pneumatikkolben 30 der Pneumatikkolben 30 aus dem Aufnahmeteil 25 herausbewegt wird.

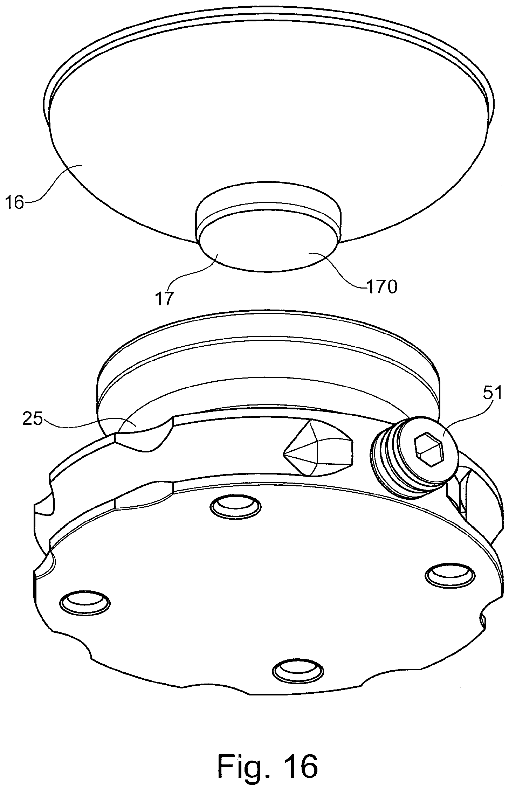

Figur 16 zeigt eine Untenansicht der Abschlusskappe 16 mit der konvexen, sphärischen Kontaktfläche 170 und der Unterseite des Aufnahmeteils 25 mit dem Auslassventil 51.

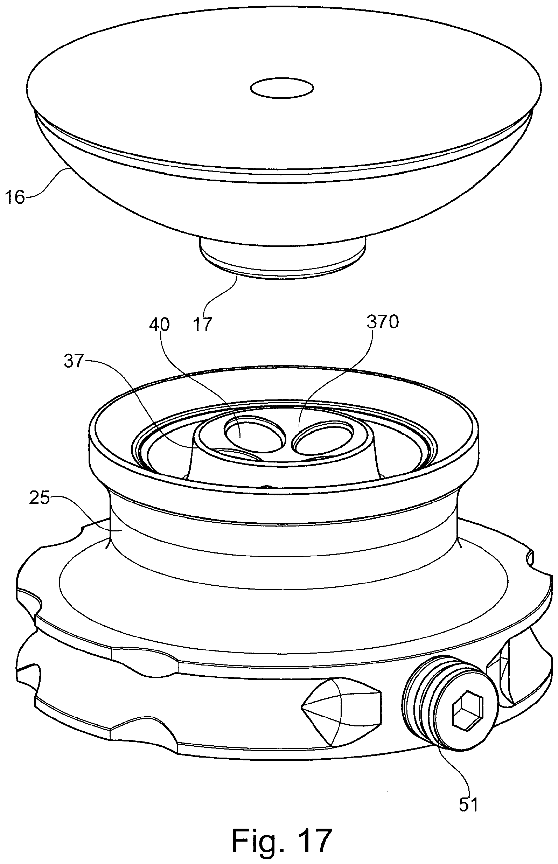

Figur 17 zeigt die Zuordnung gemäß Figur 16 in einer perspektivischen Schrägdraufsicht. Die zumindest teilweise sphärische Oberfläche 370 des proximalen Endes des Pneumatikkolbens 30 im Bereich der Befestigungseinrichtung 37 ist ebenso zu erkennen wie die zurückgesetzten Magnete 40.

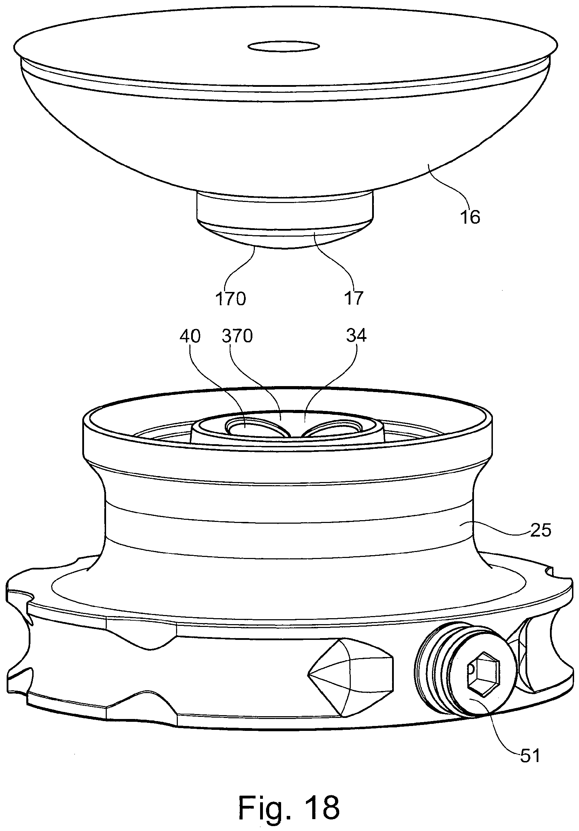

Figur 18 zeigt die Zuordnung gemäß Figur 17 in einer Schräg-Seitenansicht.

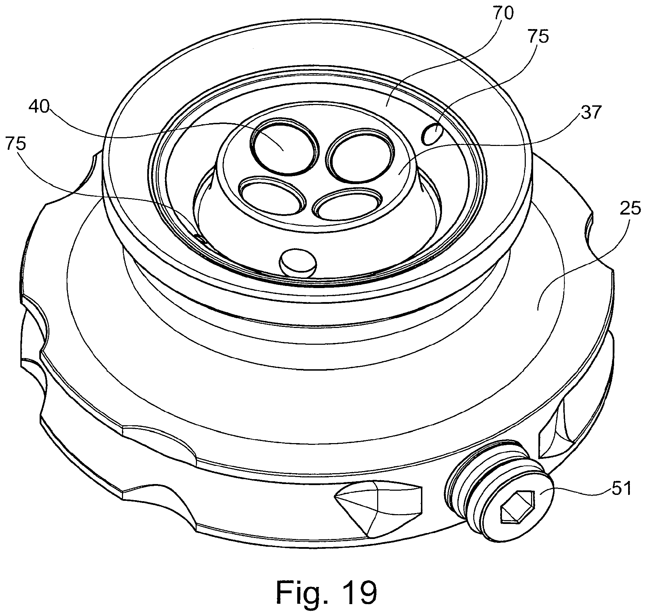

Figur 19 zeigt das Aufnahmeteil 25 in einer perspektivischen Einzeldarstellung. Es ist zu erkennen, dass die Auszugssicherung 70 zwei einander gegenüberliegende Ausnehmungen 75 aufweist, in die ein Werkzeug eingreifen kann, um die Auszugssicherung 70 an dem Aufnahmeteil 25 im Bereich der Zylinderwandung festzulegen. Neben einem Einschrauben in ein Gewinde, das in dem zylindrischen Abschnitt ausgebildet ist, wobei die Auszugssicherung 70 ein Außengewinde aufweist, kann die Auszugssicherung 70 auch über separate Schrauben oder Befestigungsmittel an dem Aufnahmeteil 25 angeschraubt oder befestigt und formschlüssig gehalten werden.

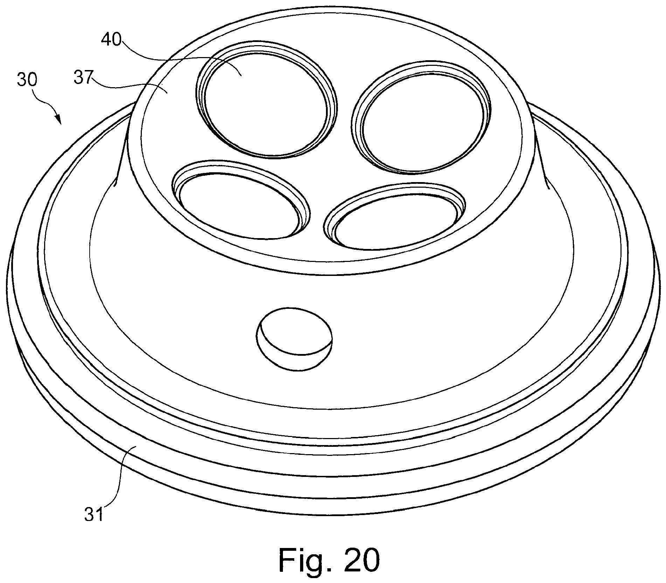

Figur 20 zeigt den Pneumatikkolben 30 in einer Einzeldarstellung mit den vier darin angeordneten Magneten, der Befestigungseinrichtung 37 mit der sphärischen Oberfläche 370 und dem radial angeordneten Dichtelement 31 in Gestalt eines Dichtringes.

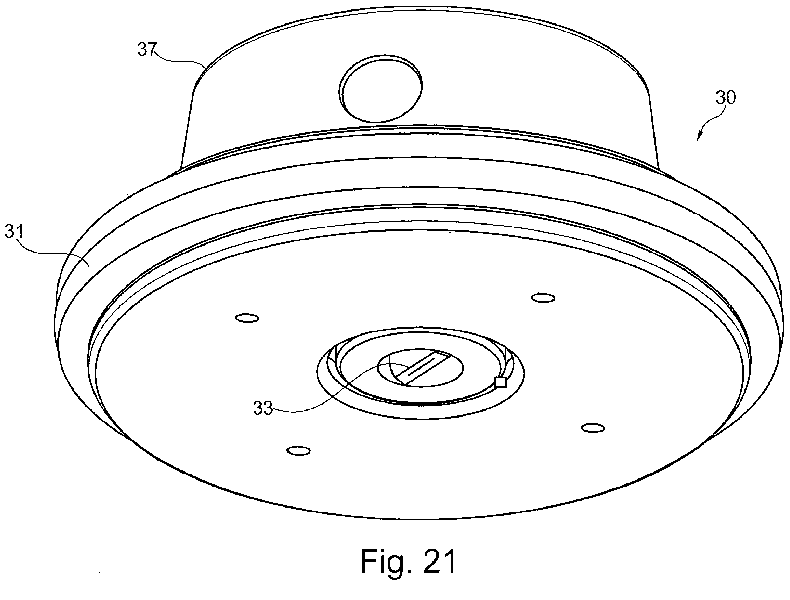

Figur 21 zeigt den Pneumatikkolben 30 in einer Untenansicht mit dem Ventil 33, das ein Einströmen von Luft in das geschlossene Volumen 50 erlaubt, ein Rückströmen jedoch verhindert. Das Ventil 33 kann als Schlauchventil oder Flatterventil ausgebildet sein und dient als Rückschlagventil.

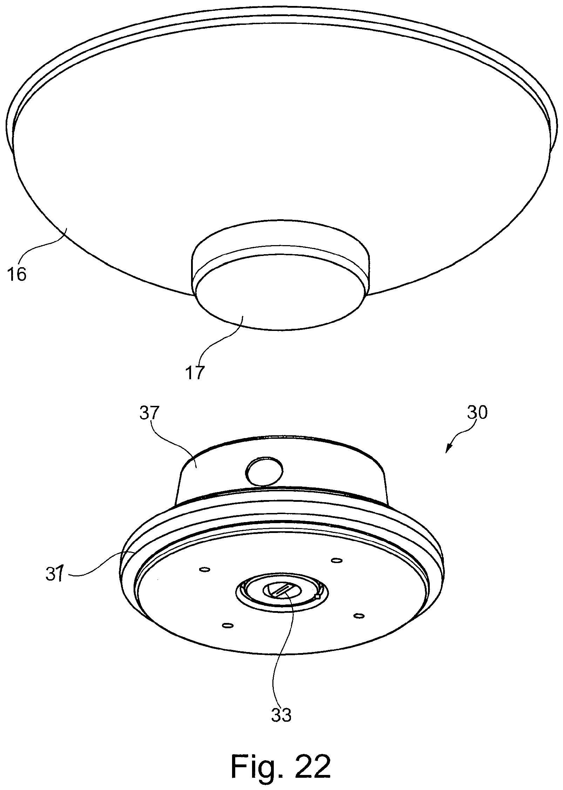

Figur 22 zeigt die Zuordnung der Abschlusskappe 16 mit dem Befestigungselement 17 als Befestigungskolben und dem Pneumatikkolben 30 von schräg unten.

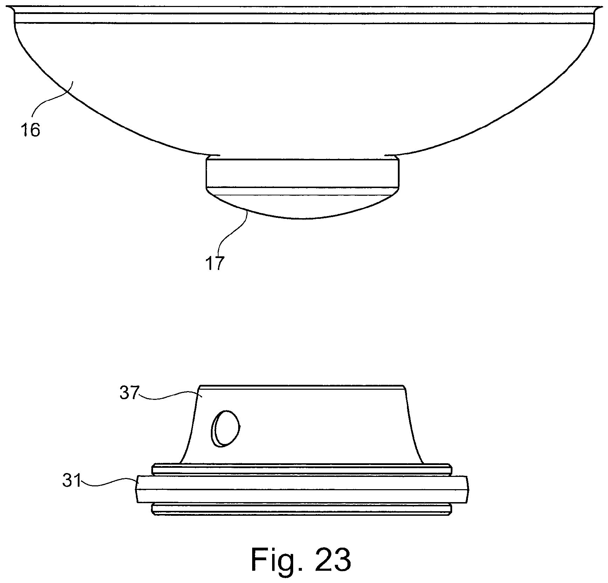

Figur 23 zeigt den Pneumatikkolben 30 mit dem Befestigungselement 37, dem Dichtring 31 vor dem Fügen mit der distalen Abschlusskappe 16.

## Patentansprüche

1. Prothesenliner (10) zur Anlage an einen Stumpf (2), mit einem elastischen Grundkörper (11), der eine proximale Öffnung (12) zum Einführen des Stumpfes (2) in einen Aufnahmeraum (13) und ein distales Ende (14) aufweist, **dadurch gekennzeichnet, dass** an der Außenseite (15) des Prothesenliners (10) zumindest ein Pneumatikkolben (30) angeordnet ist.

2. Prothesenliner nach Anspruch 1, **dadurch gekennzeichnet, dass** der Pneumatikkolben (30) ein Sperrelement (33) aufweist, das ein Rückströmen von Luft in Richtung auf den Prothesenliner (10) verhindert.

3. Prothesenliner nach Anspruch 2, **dadurch gekennzeichnet, dass** das Sperrelement (33) als Ventil oder Sperrlippe ausgebildet ist.

4. Prothesenliner nach einem der voranstehenden Ansprüche, **dadurch gekennzeichnet, dass** der Pneumatikkolben (30) beweglich an dem Prothesenliner (10) befestigt ist.

5. Prothesenliner nach einem der voranstehenden Ansprüche, **dadurch gekennzeichnet, dass** der Pneumatikkolben (30) über ein Kugelgelenk (35) oder eine elastische Anbindung an dem Prothesenliner (10) befestigt ist.

6. Prothesenliner nach einem der voranstehenden Ansprüche, **dadurch gekennzeichnet, dass** der Pneumatikkolben (30) über zumindest einen Magneten (40) an dem Prothesenliner (10) befestigt ist.

7. Prothesenliner nach Anspruch 6, **dadurch gekennzeichnet, dass** der Pneumatikkolben (30) über zumindest einen Magneten (40) konnektierbar in einem Prothesenschaftsystem aus dem Prothesenliner und einem Prothesenschaft (20) integrierbar ist.

8. Prothesenliner nach Anspruch 6 oder 7, **dadurch gekennzeichnet, dass** der Pneumatikkolben (30) zumindest bereichsweise eine sphärische Oberfläche (370) aufweist.

9. Prothesenliner nach Anspruch 6 oder 7, **dadurch gekennzeichnet, dass** an dem Prothesenliner (10) ein Befestigungselement (17) angeordnet ist, über das der Pneumatikkolben (30) an dem Prothesenliner (10) befestigt wird und das eine zu einer zumindest bereichsweise sphärischen Oberfläche (370) des Pneumatikkolbens (30) korrespondierend ausgebildete Kontaktoberfläche (170) aufweist.

10. Prothesenschaftsystem mit einem Prothesenliner (10) nach Anspruch 1 und einem Prothesenschaft (20), der mit dem Prothesenliner (10) einen Zwischenraum (60) ausbildet, **dadurch gekennzeichnet, dass** der Prothesenschaft (20) einen zu dem Pneumatikkolben (30) korrespondierenden Zylinderbereich (21) aufweist, der zur Aufnahme des Pneumatikkolbens (30) ausgebildet ist und in dem im gekoppelten Zustand des Prothesenliners (10) mit dem Prothesenschaft (20) ein geschlossenes Volumen (50) ausgebildet ist, das mit zumindest einem Sperrelement (33, 52) gekoppelt ist, das ein Rückströmen von Luft in den Zwischenraum (60) verhindert.

11. Prothesenschaftsystem nach Anspruch 10, **dadurch gekennzeichnet, dass** das geschlossene Volumen (50) mit zumindest einer Sperreinrichtung (51) in strömungstechnischer Verbindung steht, die ein Ausströmen von Luft ermöglicht und ein Rückströmen verhindert.

12. Prothesenschaftsystem nach Anspruch 10 oder 11, **dadurch gekennzeichnet, dass** das geschlossene Volumen (50) und/oder der Zwischenraum (60) mit einer Belüftungseinrichtung zum Einleiten von Luft in strömungstechnischer Verbindung steht.

13. Prothesenschaftsystem nach einem der Ansprüche 10 bis 12, **dadurch gekennzeichnet, dass** das Sperrelement (33) in dem Pneumatikkolben (30) und/oder in einer Zuleitung von dem Zwischenraum (60) zu dem geschlossenen Volumen (50) und/oder in einer Leitung von dem geschlossenen Volumen (50) zur Umgebung angeordnet ist.

14. Prothesenschaftsystem nach einem der Ansprüche 10 bis 13, **dadurch gekennzeichnet, dass** der Zylinderbereich (21) an seinem proximalen Ende eine Einführschräge (26) aufweist.

15. Prothesenschaftsystem nach einem der Ansprüche 10 bis 14, **dadurch gekennzeichnet, dass** an dem proximalen Ende des Zylinderbereiches (21) eine Auszugssicherung (70) für den Pneumatikkolben (30) angeordnet ist.

## Claims

1. A prosthetic liner (10) for application on a stump (2), comprising an elastic base body (11) which has a proximal opening (12), for insertion of the stump (2) into a receiving space (13), and a distal end (14), **characterized in that** at least one pneumatic piston (30) is arranged on the outside (15) of the prosthetic liner (10).

2. The prosthetic liner as claimed in claim 1, **characterized in that** the pneumatic piston (30) has a blocking element (33) which prevents a reverse flow of air in the direction of the prosthetic liner (10).

3. The prosthetic liner as claimed in claim 2, **characterized in that** the blocking element (33) is formed as a valve or blocking lip.

4. The prosthetic liner as claimed in one of the preceding claims, **characterized in that** the pneumatic piston (30) is movably attached to the prosthetic liner (10).

5. The prosthetic liner as claimed in one of the preceding claims, **characterized in that** the pneumatic piston (30) is attached to the prosthetic liner (10) via a ball joint (35) or an elastic connection.

6. The prosthetic liner as claimed in one of the preceding claims, **characterized in that** the pneumatic piston (30) is attached to the prosthetic liner (10) via at least one magnet (40).

7. The prosthetic liner as claimed in claim 6, **characterized in that** the pneumatic piston (30) can be connectably integrated via at least one magnet (40) into a prosthetic socket system composed of the prosthetic liner and a prosthetic socket (20).

8. The prosthetic liner as claimed in claim 6 or 7, **characterized in that** the pneumatic piston (30) has an at least partially spherical surface (370).

9. The prosthetic liner as claimed in claim 6 or 7, **characterized in that** an attachment element (17) is arranged on the prosthetic liner (10), via which the pneumatic piston (30) is attached to the prosthetic liner (10) and which has a contact surface (170) formed to correspond to an at least partially spherical surface (370) of the pneumatic piston (30).

10. A prosthetic socket system having a prosthetic liner (10) according claim 1 and a prosthetic socket (20) which forms an intermediary space (60) with the prosthetic liner (10), **characterized in that** the prosthetic socket (20) has a cylinder region (21) corresponding to the pneumatic piston (30), which cylinder region (21) is formed to receive the pneumatic piston (30) and in which a closed volume (50) is formed in the coupled state of the prosthetic liner (10) with the prosthetic socket (20), which closed volume (50) is coupled to at least one blocking element (33, 52) which prevents a reverse flow of air into the intermediary space (60).

11. The prosthetic socket system as claimed in claim 10, **characterized in that** the closed volume (50) has a fluidic connection to at least one blocking mechanism (51), which enables an outflow of air and prevents a reverse flow.

12. The prosthetic socket system as claimed in claim 10 or 11, **characterized in that** the closed volume (50) and/or the intermediary space (60) are fluidically connected to a ventilation mechanism for the introduction of air.

13. The prosthetic socket system as claimed in one of claims 10 to 12, **characterized in that** the blocking element (33) is arranged in the pneumatic piston (30) and/or in a supply line from the intermediary space (60) to the closed volume (50) and/or in a line from the closed volume (50) to the environment.

14. The prosthetic socket system as claimed in one of claims 10 to 13, **characterized in that** the cylinder region (21) has an insertion bevel (26) at its proximal end.

15. The prosthetic socket system as claimed in one of claims 10 to 148, **characterized in that** a pull-out safety (70) for the pneumatic piston (30) is arranged at the proximal end of the cylinder region (21).

## Revendications

1. Manchon prothétique (10) destiné à être appliqué sur un moignon (2), comportant un corps de base élastique (11) qui présente une ouverture proximale (12) destinée à l'introduction du moignon (2) dans un espace de réception (13) et une extrémité distale (14),
**caractérisé en ce que**
au moins un piston pneumatique (30) est agencé sur la face extérieure (15) du manchon prothétique (10).

2. Manchon prothétique selon la revendication 1,
**caractérisé en ce que**
le piston pneumatique (30) comprend un élément d'arrêt (33) qui empêche un reflux d'air en direction vers le manchon prothétique (10).

3. Manchon prothétique selon la revendication 2,
**caractérisé en ce que**
l'élément d'arrêt (33) est réalisé sous la forme d'une valve ou d'une lèvre d'arrêt.

4. Manchon prothétique selon l'une des revendications précédentes,
**caractérisé en ce que**
le piston pneumatique (30) est fixé de façon mobile au manchon prothétique (10).

5. Manchon prothétique selon l'une des revendications précédentes,
**caractérisé en ce que**
le piston pneumatique (30) est fixé au manchon prothétique (10) par une articulation à rotule (35) ou par une attache élastique.

6. Manchon prothétique selon l'une des revendications précédentes,
**caractérisé en ce que**
le piston pneumatique (30) est fixé au manchon prothétique par au moins un aimant (40).

7. Manchon prothétique selon la revendication 6,
**caractérisé en ce que**
le piston pneumatique (30) est susceptible d'être intégré dans un système à emboîture de prothèse, constitué par le manchon prothétique et par une emboîture de prothèse (20), en étant connecté par un aimant (40).

8. Manchon prothétique selon la revendication 6 ou 7,
**caractérisé en ce que**
le piston pneumatique (30) présente au moins localement une surface sphérique (370).

9. Manchon prothétique selon la revendication 6 ou 7,
**caractérisé en ce que**
un élément de fixation (17) est agencé sur le manchon prothétique (10), élément via lequel le piston pneumatique (30) est fixé au manchon prothétique (10) et qui présente une surface de contact (170) réalisée de façon correspondante à une surface (370) au moins localement sphérique du piston pneumatique (30).

10. Système à emboîture de prothèse comportant un manchon prothétique (10) selon la revendication 1 et une emboîture de prothèse (20) qui réalise un espace intermédiaire (60) avec le manchon prothétique (10),
**caractérisé en ce que**
l'emboîture de prothèse (20) présente une zone cylindrique (21) correspondant au piston pneumatique (30), laquelle est réalisée pour recevoir le piston pneumatique (30) et dans laquelle, dans l'état couplé du manchon prothétique (10) avec l'emboîture de prothèse (20), réalise un volume fermé qui est couplé à au moins un élément d'arrêt (33, 52) qui empêche un reflux d'air vers l'espace intermédiaire (60).

11. Système à emboîture de prothèse selon la revendication 10,
**caractérisé en ce que**
le volume fermé (50) est en communication fluidique avec au moins un moyen d'arrêt (51) qui permet à l'air de sortir et qui empêche un écoulement en reflux.

12. Système à emboîture de prothèse selon la revendication 10 ou 11,
**caractérisé en ce que**
le volume fermé (50) et/ou l'espace intermédiaire (60) est en communication fluidique avec un moyen de ventilation pour introduire de l'air.

13. Système à emboîture de prothèse selon l'une des revendications 10 à 12,
**caractérisé en ce que**
l'élément d'arrêt (33) est agencé dans le piston pneumatique (30) et/ou dans une conduite d'amenée depuis l'espace intermédiaire (60) vers le volume fermé (50) et/ou dans une conduite depuis le volume fermé (50) vers l'environnement.

14. Système à emboîture de prothèse selon l'une des revendications 10 à 13,
**caractérisé en ce que**
la zone cylindrique (21) présente un chanfrein d'introduction (26) à son extrémité proximale.

15. Système à emboîture de prothèse selon l'une des revendications 10 à 14,
**caractérisé en ce que**
un moyen de blocage anti-extraction (70) pour le piston pneumatique (30) est agencé à l'extrémité proximale de la zone cylindrique (21).
